# EUROPEAN PATENT SPECIFICATION

(11) **EP 4 103 072 B1**
(45) Date of publication and mention of the grant of the patent: **30.04.2025**
(21) Application number: 20728003.3
(22) Date of filing: 22.05.2020
(51) Int. Cl.: A61B 17/12, A61B 90/00

(54) **EMBOLISATION DEVICES AND METHODS OF MANUFACTURING THE SAME**
EMBOLISATIONSVORRICHTUNGEN UND VERFAHREN ZUR HERSTELLUNG DAVON
DISPOSITIFS D'EMBOLISATION ET LEURS PROCÉDÉS DE FABRICATION

(43) Date of publication of application: 21.12.2022
(73) Proprietor: Clearstream Technologies Limited, Enniscorthy, County Wexford (IE)
(72) Inventor: RONAN, Allan, Enniscorthy, County Wexford (IE); SHERIDAN, Stephen, Enniscorthy, County Wexford (IE); WALL, Seán, Enniscorthy, County Wexford (IE); GILES, Ciaran, Enniscorthy, County Wexford (IE)
(74) Representative: Hoffmann Eitle
(86) International application number: PCT/EP2020/064297
(87) International publication number: WO 2021/233553

(56) References cited:
- WO-A2-2005/113035
- US-A1- 2015 257 765
- US-A1- 2016 296 238
- US-A1- 2020 078 022

## Description

### Technical Field

The present disclosure generally relates to embolisation devices for promoting clot formation in a bodily lumen, having a collapsed delivery configuration and an expanded deployed configuration. The present disclosure also relates to a method of manufacturing the embolisation device, and a manufacturing device for manufacturing the embolisation device.

### Background

Embolisation devices may be deployed in the vasculature at a particular location by a medical practitioner so as to promote blood clot formation and ultimately occlude the blood vessel. Typically, an embolisation device is pushed through a guide catheter using a delivery wire until a point of deployment is reached. Once the device reaches the required point of deployment, the device is deployed from the guide catheter in a distal direction, detached from the delivery wire, and the delivery wire and delivery catheter are removed.

It is important that the embolisation device does not migrate within the vasculature once deployed, as this could cause serious adverse effects. Likewise, it is important that the constituent components of the embolisation device do not break, detach or become dislodged from the embolisation device as these could also migrate within the vasculature, causing similar adverse effects.

Accordingly, there is a need for embolisation devices with a reduced risk of migration.

There is also a need for embolisation devices to have a lesser longitudinal extent such that they are able to be deployed in a wider variety of vasculatures.

US 2015/257765 A1 discloses an occlusion device for a vascular or biological lumen including a plurality of coiling members held together at both the proximal and distal ends by retaining features. A restraining loop holds coiling members together at a point along a length of the coiling members.

US 2016/296238 A1 discloses a vascular filter including a hub and a basket portion. The filter is formed from a plurality of strands of a biocompatible material such as a polymer or a super-elastic metal alloy. The strands are intertwined to form the hub. The strands are then secured by forming a knot such as a crown knot or a splice. The free ends of the strands form a basket portion of the filter.

### Summary

According to a first aspect, there is provided an embolisation device for promoting clot formation in a bodily lumen, the embolisation device having a collapsed delivery configuration and an expanded deployed configuration, the embolisation device comprising a plurality of flexible bristles, wherein at least one of the plurality of bristles comprises a twisted portion, the at least one twisted portion securing the plurality of bristles to one another, and wherein the plurality of bristles extend at least radially outwardly from the at least one twisted portion. The one or more twisting portions may inhibit the individual bristles from becoming detached from the embolisation device. The twisting portions may negate the need for a separate securing element for securing the bristles together, such as a stem, and reduce the longitudinal extent of the embolisation device, allowing the device to be deployed in smaller or shorter vasculatures.

The embolisation device of the first aspect further comprises a core body, wherein the at least one twisted portion of the bristles are located within the core body such that the core body surrounds and secures the at least one twisted portion. The core body may further inhibit the bristles from becoming detached from the embolisation device.

The core body of the embolisation devices disclosed herein may mechanically anchor the plurality of flexible bristles.

The core body may adhere or bond to the plurality of flexible bristles.

The core body may be a curable material or settable material, and may be curable or settable upon heating, solvent flashing and/or irradiating.

The core body may be cured or set such that the core body surrounds and secures the portion of the flexible bristle.

The core body may be a polymer, and, optionally, a nylon. The core body may be a resin. The core body may be a metal and/or an alloy.

According to a second aspect, there is provided an embolisation system comprising an embolisation device of the first aspect, further comprising an interconnecting module attached to the embolisation device at the core. The interconnecting module may be configured to connect to a delivery element. The interconnecting module may be configured to connect to a bristle segment comprising a second plurality of bristles. The bristle segment may comprise a plurality of bristles secured in the same manner as the first aspect, or may comprise a plurality of bristles extending at least radially outwardly from a stem.

The embolisation system may further comprise a radiopaque marker attached to the core.

According to a third aspect, there is provided an embolisation device for promoting clot formation in a bodily lumen, the embolisation device having a collapsed delivery configuration and an expanded deployed configuration, the embolisation device comprising a plurality of bristle segments, each segment comprising a plurality of flexible bristles, wherein, for at least one of the bristle segments: at least one of the plurality of bristles comprises a twisted portion, the at least one twisted portion securing the plurality of bristles in the segment to one another, and wherein the plurality of bristles extend at least radially outwardly from the at least one twisted portion. The embolisation device of the third aspect further comprises a core body, wherein the at least one twisted portion of the bristles are located within the core body such that the core body surrounds and secures the at least one twisted portion.

According to a fourth aspect, there is provided method of manufacturing an embolisation device for promoting clot formation in a lumen and having a collapsed delivery configuration and an expanded deployed configuration, the method comprising: providing a plurality of flexible bristles; and twisting at least one bristle of the plurality of bristles to form at least one twisted portion, the at least one twisted portion securing the plurality of bristles to one another such that the bristles extend at least radially outwardly from the at least one twisted portion. The method comprises providing a core body, wherein the at least one twisted portion of the bristles are located within the core body such that the core body surrounds and secures the at least one twisted portion.

### Brief Description of the Drawings

For a better understanding of the present disclosure, and to show how the same may be carried into effect, reference will be made, by way of example only, to the following schematic drawings, in which:
Figure 1A shows a side view of an embolisation device in an unconstrained configuration according to one or more examples not falling within the scope of the appended claims;
Figure 1B shows a cross-sectional side view of the embolisation device of Figure 1A, according to one or more examples not falling within the scope of the appended claims;
Figure 2A shows a side view of an embolisation device in a collapsed delivery configuration inside a delivery catheter, according to one or more examples not falling within the scope of the appended claims;
Figure 2B shows a side view of an embolisation device in an expanded deployed configuration inside a bodily lumen according to one or more examples not falling within the scope of the appended claims;
Figure 3 shows a side view of another embolisation device in an unconstrained configuration, according to one or more examples not falling within the scope of the appended claims;
Figure 4 shows a side view of yet another embolisation device in an unconstrained configuration, according to one or more embodiments shown and described herein;
Figure 5 shows a side view of a further embolisation device in an unconstrained configuration, according to one or more embodiments shown and described herein;
Figure 6A shows a side view of an embolisation device in a collapsed delivery configuration inside a delivery catheter, according to one or more embodiments shown and described herein; and
Figure 6b shows a side view of an embolisation device in an expanded deployed configuration inside a delivery catheter, according to one or more embodiments shown and described herein.

### Detailed Description

Throughout this disclosure the term 'embolisation device' may refer to a device which may be permanently or semi-permanently implanted in a bodily lumen. Accordingly, the 'embolisation device' may be configured to be disposed within the bodily lumen for a period of time, such as a number of days, or disposed in the lumen indefinitely. To this end, the 'embolisation device' may be configured to be selectively detached from a delivery element so that it may be implanted in the bodily lumen in isolation.

Throughout this disclosure the term 'bodily lumen' may refer to the inside space within a tubular structure of the human or animal body. The 'bodily lumen' may be, for example, an artery or vein.

Throughout this disclosure the term 'collapsed delivery configuration' of an element may refer to a configuration of the element which has a smaller radial extent than an expanded deployed configuration of the element.

Throughout this disclosure the term 'to anchor' may refer to partly or fully securing an element in a position.

Throughout this disclosure, the term 'stem' may refer to an elongate element which extends longitudinally along the length of the embolisation device to act as a backbone for the device, and has a significantly smaller radial extent than the further elements of the embolisation device (for example, the plurality of flexible bristles). A stem may extend along substantially the whole longitudinal extent of the plurality of flexible bristles (e.g. when the embolisation device is in an unrestrained configuration, collapsed delivery configuration and/or expanded deployed configuration). The stem may extend along substantially the whole length of the embolisation device.

Throughout this disclosure, the term 'core body' may refer to any body suitable for surrounding and securing a portion of a flexible bristle which extends through the core body.

In any of the examples described herein, the term 'bristle' may refer to an elongate strand of material formed substantially a single piece. The 'bristle' may be a resilient bristle. The resilient bristle may be biased towards a particular curvature.

Throughout this disclosure, the term 'radially outwardly' does not exclude the element additionally extending in the longitudinal direction of the device. For example, the plurality of flexible bristles may extend radially outwardly and longitudinally from the core body or longitudinal axis of the embolisation device.

Through this disclosure, the term 'radial extent' may refer to a radial size in a particular direction radially outwardly from the centre of the core body or twisted portion of the embolisation device. For example, the embolisation device has a lower radial extent in the collapsed delivery configuration than in the expanded deployed configuration.

Throughout this disclosure, the term 'surround' may refer to when a first element contacts a second element about substantially an entire circumference of the second element.

Throughout this disclosure, the term 'mechanically anchor' refers to the anchoring of an element substantially by mechanical forces caused by the macroscopic properties of the anchoring element, rather than intermolecular forces and/or chemical bonds between the anchoring element and the anchored element which are responsible for adhering/bonding.

Throughout this disclosure, the term "bristle segment" may refer to a group of bristles wherein the spacing between adjacent bristles is less than a predetermined distance. When two bristle segments are "spaced apart", the spacing between the bristle segments (i.e. the spacing between the most distal bristle of the first segment and the most proximal bristle of the second segment) is greater than the spacing between adjacent bristles within at least one of the bristle segments.

The plurality of flexible bristles may have a collapsed configuration in the collapsed delivery configuration. The plurality of flexible bristles may have an expanded configuration in the expanded deployed configuration. The plurality of bristles may extend radially outwardly from the stem in a plurality of radial directions about the stem.

In the expanded configuration, the plurality of flexible bristles may be configured to anchor the device in the bodily lumen. The plurality of flexible bristles may be configured to provide substantially all of the anchoring force for the embolisation device in the bodily lumen.

In the expanded configuration, the plurality of flexible bristles may be configured to contact the bodily lumen.

The collapsed delivery configuration is adopted when the device is positioned inside the delivery catheter. More particularly, in the collapsed delivery configuration, the plurality of flexible bristles extending outwardly from the stem have a radial extent which is less than the radial extent of the bristles in the expanded deployed configuration of the element.

The bristles may be made of a flexible or resiliently deformable material such as stainless steel, Elgiloy or Nitinol. Other suitable materials may also be used, such as any suitable polymer or any other shape memory metal or metal alloy.

The diameter of an individual flexible bristle may range from 0.036mm (0.0014 inches) to 0.053mm (0.0021 inches). For example, the diameter of an individual flexible bristle may be 0.0381mm (0.0015 inches), 0.0445mm (0.00175 inches) or 0.0508mm (0.002 inches). The average radial diameter of the radial profile formed by the expanded flexible bristles may range from 5mm to 30mm.

A flow restrictor may be a membrane made from thin film Nitinol, thin film PTFE, a thin film elastomer such as polyurethane, a polymer or any other type of suitable biocompatible material. The membrane may be made of any self-expanding material. The membrane may have a thickness of 4µm to 35µm and a radial diameter of 5mm to 20mm. For example, the diameter of the membrane may be 6.5mm, 9mm or 16mm. Furthermore, the membrane may be non-permeable or semi-permeable.

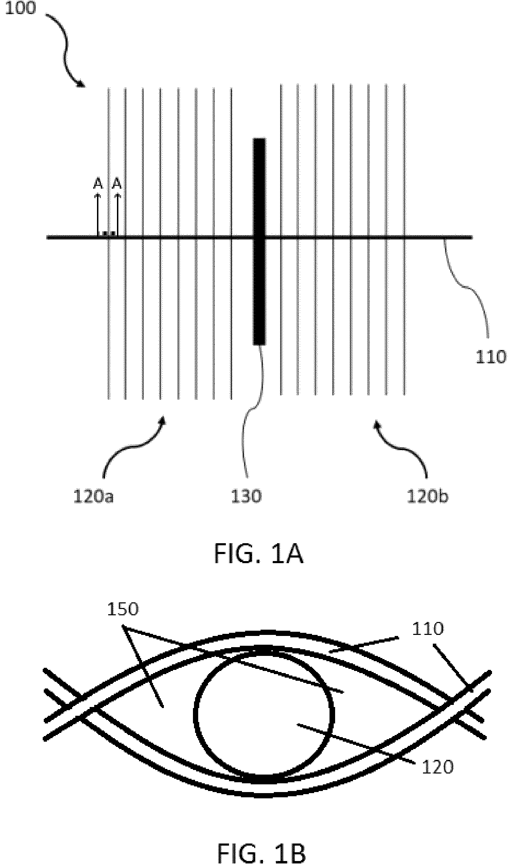

Figure 1A schematically depicts an embolisation device 100. The embolisation device 100 is configured for deployment in a bodily lumen so as to promote clot formation therein. The embolisation device 100 in Figure 1 is shown in an unconstrained configuration.

The embolisation device 100 comprises a stem 110, a plurality of flexible bristles 120 in bristle segments 120a, 120b and a flow restricting membrane 130.

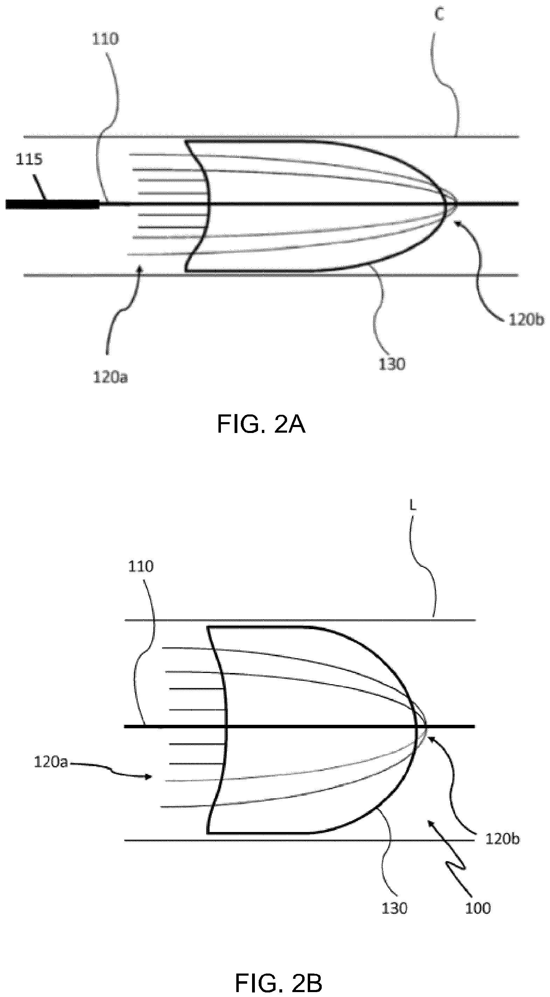

The embolisation device 100 is guided through a delivery catheter using a delivery element 115 (depicted schematically in Figure 2A). The delivery element may be releasably connected to a proximal end of the embolisation device, for example by a threaded connection. Once the embolisation device has been delivered to the target site within the bodily lumen, then the embolisation device is moved distally relative to the delivery catheter such that the bristles expand to the expanded deployed configuration and engage the walls of the bodily lumen to anchor the device to the lumen. The embolisation device 100 is then released from the delivery element and the delivery element and delivery catheter are withdrawn.

The stem 110 of the embolisation device 100 may be made of a twisted wire or wires, wherein the twisted wire holds the bristles 120 in the embolisation device. Figure 1B shows close up cross-section view of the embolisation device 100, taken along the line A-A in Figure 1A. The bristle 120 shown in Figure 1B extends out of the plane of the figure and is shown in cross section. As can be seen by Figure 1B, the twisted wire of the stem 110 abuts or clamps the bristle 120 at two radially opposite locations on the surface of the bristle. The friction provided by this contact between the stem 110 and the bristle 120 holds the bristle 120 in place. However, in the illustrated example, the twisted wire of the stem 110 does not surround the part of the bristle 120 that extends between the twisted wires. In particular, the twisted wire only contacts the bristle 120 at two points, rather than about the circumference of the part of the bristle 120 between the twisted wires. This is indicated by the gaps 150 in Figure 1B. In other words, the frictional force applied by the twisted wires to the bristle 120 are centred upon only two points on the surface of the bristle 120. As a result, there remains a risk that the bristle 120 detaches or dislodges from the twisted wire and from the embolisation device 100. Furthermore, it has been observed that once a single bristle becomes dislodged from the embolisation device 100, the force required to dislodge further bristles is greatly reduced. As such, the risk of further bristles becoming dislodged increases.

The stem 110 may also increase the longitudinal dimension of the embolisation device, meaning that some vasculatures may not be suitable for the embolisation device 100 as it is too long.

Figure 2A schematically depicts a side view of an embolisation device 100 in a collapsed delivery configuration inside a delivery catheter C.

As can be seen from Figure 2A, in the collapsed delivery configuration of the embolisation device 100, the plurality of flexible bristles 120a, 120b have been collapsed into a collapsed configuration.

As also can be seen from Figure 2A, in the collapsed delivery configuration of the embolisation device 100, the flow restricting membrane 130 has been collapsed into a collapsed configuration.

In Figure 2A, both the proximal bristle segment 120a and the distal bristle segment 120b point proximally. However, as will be evident to the person skilled in the art, any arrangement in this regard is possible. In particular, the proximal bristle segment 120a and the distal bristle segment 120b may point distally. The proximal bristle segment 120a may point proximally and the distal bristle segment 120b may point distally. The proximal bristle segment 120a may point distally and the distal bristle segment 120b may point proximally.

Figure 2B schematically depicts the embolisation device 100 in an expanded deployed configuration in a bodily lumen L. The embolisation device 100 may be pushed through the delivery catheter C by delivery element 115 (for example a delivery wire) until it reaches the distal end of the catheter C. Once the end of the catheter is in the target position in a bodily lumen L, the device 100 is moved distally relative to the catheter C by the delivery element 115, with the catheter C held in place. The device 100 is then disposed within the bodily lumen L in the expanded deployed configuration by removing the delivery catheter C whilst inserted in the bodily lumen L such that the embolisation device 100 is allowed to expand in the bodily lumen L. The delivery element 115 is then removed from the device 100, for example by twisting the delivery element to cause the two to detach.

The expanded deployed configuration of the embolisation device 100 has a greater radial extent than the collapsed delivery configuration shown in Figure 2A. In the expanded deployed configuration shown in Figure 2B, the plurality of flexible bristles 120a, 120b and the flow restricting membrane 130 contact the bodily lumen L so as to anchor the embolisation device 100 within the bodily lumen L. The anchoring force provided by the plurality of flexible bristles 120a, 120b and the flow restricting membrane 130 may be sufficient to resist migration of the embolisation device 100 in the bodily lumen L.

In the expanded deployed configuration shown in Figure 2B, the embolisation device 100 may occlude the bodily lumen L and promote clot formation therein.

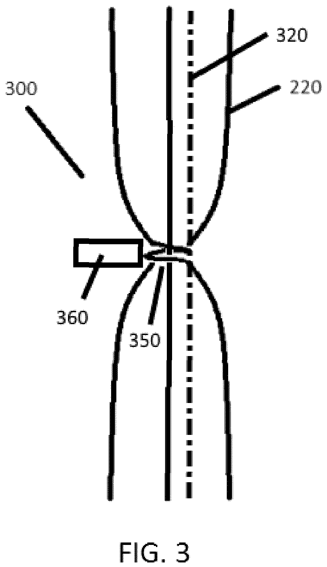

Figure 3 schematically depicts another embolisation device 300 according to the present disclosure. Again, the embolisation device 300 is configured for deployment in a bodily lumen so as to promote clot formation therein. The embolisation device 300 in Figure 3 is shown in an unconstrained configuration.

The embolisation device 300 comprises a plurality of bristles 220. At least one of the plurality of bristles 220 comprises a twisted portion 350. The twisted portion secures the plurality of bristles to one another, such that the plurality of bristles are secured together by the twisted portion 350. For example, the at least one twisted portion may be threaded about the other bristles in a manner that is sufficient to secure them together. It is noted that the twisted portion 350 is simplified for illustration purposes. It will be apparent to the skilled person that there are many different possible configurations of the at least one twisted portion that can be used to secure the plurality of bristles 220 together, such as one or more knots or loops or any combination thereof. In other examples, a plurality of bristles 220 may have a twisted portion to ensure that the bristles 220 are securely fastened together. In some examples, the plurality of bristles may be plaited or braided to one another. As the bristles 220 are secured to one another by the twisted portion 350, the longitudinal extent of the embolisation device 300 is reduced as a separate securing element is not required. For example, a longitudinally extending stem upon which the bristles are secured may not be required.

The embolisation device 300 may further comprise a flow restrictor, such as a flow restricting membrane 320 (for example a disc-shaped membrane), located longitudinally within the bristles. The flow restrictor may be mounted on the device by manipulating a portion of bristles such that they extend longitudinally in a low radial profile (for example using a bristle manipulator), mounting the flow restrictor over the portion of bristles onto the device (i.e. passing them through a central hole of the membrane), and releasing the bristles such that the flow restrictor is positioned longitudinally within the bristles and secured in place by the proximal and distal neighbouring bristles, as shown in Figure 3. Alternatively, the membrane 220 may be a mesh membrane and at least some of the bristles pass through holes in the mesh proximal to the centre of the mesh membrane such that those bristles secure the membrane 220 to the device 300.

As in the case of the embolisation device 200 shown in Figures 2A and 2B, the embolisation device 300 has a collapsed delivery configuration in which it may be placed within a delivery catheter, analogous to the configuration shown in Figure 2B. The embolisation device 300 may be pushed through a delivery catheter by a delivery element (e.g. as shown in Figure 2A, for example by a push wire or delivery wire), which may be releasably connected to the embolisation device by an interconnecting module 360 comprising a releasable connecting mechanism, or which may be a pushing element. Once the embolisation device is at the desired location within a bodily lumen, the embolisation device 300 and delivery catheter are moved relative to one another and the embolisation device 300 delivered to the bodily lumen. For example, the catheter is retracted whilst the delivery element is held in place. The delivery element is then released from the embolisation device 300 (if necessary) and removed from the bodily lumen with the delivery catheter.

The connecting mechanism may be any suitable connecting mechanism. For example, the mechanism may comprise a male/female threaded connector connected to the embolisation device, and a female/male threaded connector connected to the delivery element. The delivery element may then be rotated to release the embolisation device 200 from the delivery element. It will be apparent to the skilled person that any other releasable connecting mechanism would be suitable. The interconnecting module 360 comprising the connecting mechanism may be connected to the embolisation device 300 by adhesive or welding.

In the expanded deployed configuration, the bristles 220 of the embolisation device 300 extend outwardly to engage the bodily lumen to anchor the device to the bodily lumen, analogous to the configuration shown in Figure 2B.

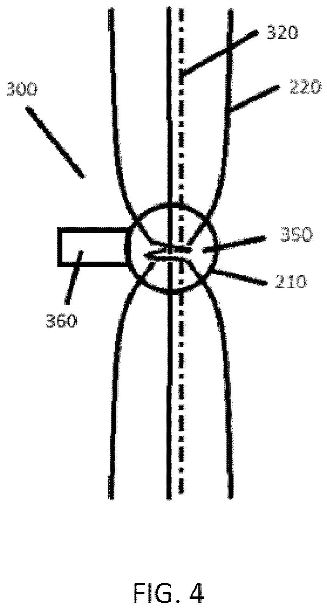

Figure 4 schematically depicts another embolisation device 300 according to the present disclosure. Again, the embolisation device 300 is configured for deployment in a bodily lumen so as to promote clot formation therein. The embolisation device 300 in Figure 4 is shown in an unconstrained configuration.

As in the case of the embolisation device shown in Figure 3, the embolisation device 300 comprises a plurality of bristles 220. At least one of the plurality of bristles 220 comprises a twisted portion 350. The twisted portion secures the plurality of bristles to one another, such that the plurality of bristles are secured together by the twisted portion 350, as described above in relation to Figure 3. It will again be apparent that there are many different possible configurations of the twisted portion that can be used to secure the plurality of bristles 220 together. In other examples, a plurality of bristles 220 may each have a twisted portion to ensure that the bristles 220 are securely fastened together. In some examples, the plurality of bristles may be plaited or braided to one another.

In addition to the twisted portion 350, the embolisation device 300 of Figure 4 comprises a core body 210 surrounding and securing the twisted portion 350. The twisted portion 350 and the core body 210 both work to inhibit the flexible bristles 220 from become dislodged. The bristles comprising the twisted portions are especially inhibited from being dislodged (e.g. extracted) from the core body 210 due to the presence of the twisted portion. In embodiments where the bristles are plaited or braided together, the plaits inhibit each of the bristles from becoming dislodged.

The core body 210 of Figure 4 may also be made of any suitable material for surrounding and securing the portions of the flexible bristles extending through the core body. For example, the core body 210 may be made of a material that adheres or bonds to the plurality of flexible bristles. Additionally and alternatively, the cored body 210 may be configured such that the core body 210 mechanically anchors the flexible bristles 220.

For example, the core body 210 may be a curable material or settable material, for example which is curable or settable upon heating, solvent flashing and/or irradiating. The core body 210 may be a polymer, a nylon, a resin or a metal or metal alloy.

The device 300 may also comprise a flow restrictor corresponding to the previous embodiments. The flow restrictor may comprise a membrane 320, for example a disc-shaped membrane, and may be mounted on the device by manipulating a portion of the bristles to extend longitudinally in a low radial profile, passing them through a central hole of the membrane, and releasing the bristles so that the membrane is secured longitudinally between the bristles. The flow restrictor may also extend partially through the core body 350 to further secure the membrane 320 to the device. For example, the membrane 320 may be mounted to the device before the core body 350 is added to the device during manufacture.

The embolisation device 300 of Figure 4 again has a collapsed delivery configuration in which it may be placed within a delivery catheter C. The embolisation device 300 may be pushed through a delivery catheter by a delivery element, which may be releasably connected to the embolisation device by a connecting mechanism. Once the embolisation device is at the desired location within a bodily lumen, the embolisation device 300 and the delivery catheter are moved relative to one another and the embolisation device is delivered to the bodily lumen. For example, delivery element is held in place and the catheter is retracted. The delivery element is then released from the embolisation device 300 and removed from the bodily lumen with the delivery catheter.

The connecting mechanism may be any suitable connecting mechanism such as a screw mechanism, and may be comprised in an interconnecting module 360 connected to the device as discussed in further detail in the previously-described examples. For example, the module 360 may be secured to the device 300 by the core body 350 or by adhesive or welding to the core body 350.

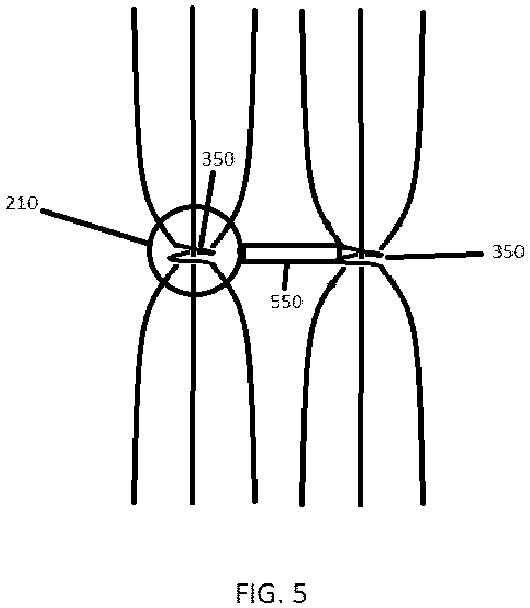

Figure 5 schematically depicts another embolisation device, according to the present disclosure, in an unconstrained configuration. The embolisation device of Figure 5 comprises two bristle segments, each comprising a plurality of bristles extending generally (i.e. at least) radially outwardly from a core body 210 and from a twisted portion 350, respectively. The two bristle segments are connected to one another. The bristle segments may be directly connected to one another, for example via adhesive or welding, or spaced apart via an interconnecting module 550, which may be attached to the respective bristle segments by, for example, welding or adhesive. The interconnecting module 550 may be made of any biocompatible material, and may be a polymer, nylon, a resin, a metal or an alloy. The interconnecting module may be a unitary body. A flow restrictor may be mounted between the bristle segments (e.g. on the interconnecting module 550) or between the bristles of a bristle segment. As before, the embolisation device shown in Figure 5 has a collapsed delivery configuration in which it may be placed in a delivery catheter, and may be deployed to a bodily lumen in the same manner as with the previously described examples (i.e. an interconnecting module comprising a releasable connecting mechanism may be attached to a proximal end of the device for releasably connecting the device to a delivery element).

It will be apparent to the skilled person that the interconnecting module 550 may be configured to connect any two of the embolisation devices disclosed herein, and an embolisation device of any number of spaced apart bristle segments may be provided by using a plurality of interconnecting modules 550 to connect them.

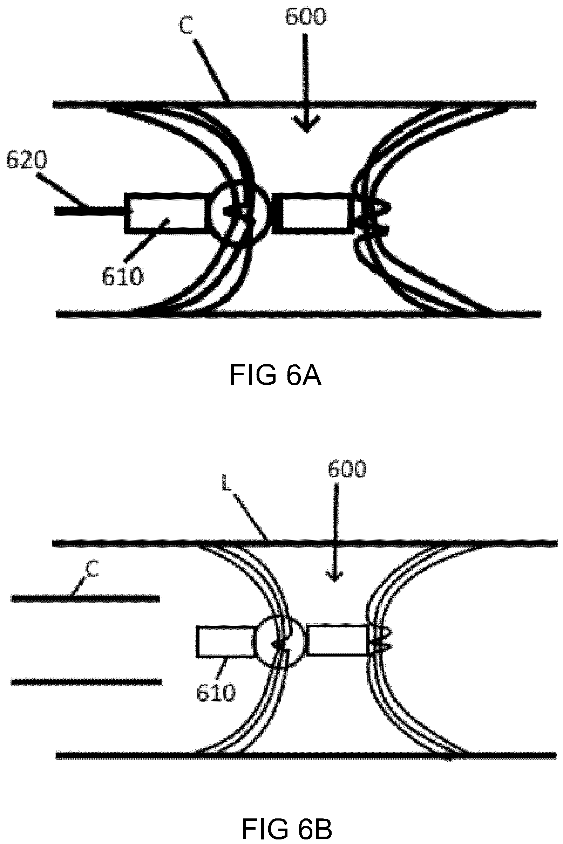

Figure 6A schematically depicts a side view of an embolisation device 600 in a collapsed delivery configuration inside a delivery catheter C. The embolisation device 600 may be any of the embodiments disclosed here, for example the embodiments described with reference to Figures 3, 4 or 5.

As can be seen from Figure 6A, in the collapsed delivery configuration of the embolisation device 600, the plurality of flexible bristles and (optionally) the flow restrictor of the device have been collapsed into a collapsed configuration. It is noted, as with the example shown in Figure 2A, that the flexible bristles of each segment of the device 600 may point in either the proximal or the distal direction when in the collapsed configuration.

Figure 6B schematically depicts the embolisation device 600 in an expanded deployed configuration in a bodily lumen L. The embolisation device 600 may be pushed through the delivery catheter C by delivery element 620 (for example a delivery wire) until it reaches the distal end of the catheter C. Once the end of the catheter is in the target position in a bodily lumen L, the device 600 is moved distally relative to the catheter C by the delivery element 620, with the catheter C held in place. The device 600 is then disposed within the bodily lumen L in the expanded deployed configuration by removing the delivery catheter C whilst inserted in the bodily lumen L such that the embolisation device 600 is allowed to expand in the bodily lumen L. The delivery element 620 is then removed from the device 600, for example by twisting the delivery element to cause the two to detach when the delivery element 620 detaches from an interconnecting module 610 of the device 600. Alternatively, the device may not comprise an interconnecting module 610 and the delivery element 620 may be a push wire. Accordingly, the device 600 is then delivered by simply pushing the device 600 out of the catheter C.

The expanded deployed configuration of the embolisation device 600 has a greater radial extent than the collapsed delivery configuration shown in Figure 6A. In the expanded deployed configuration shown in Figure 6B, the plurality of flexible bristles and (optionally) the flow restricting membrane contact the bodily lumen L so as to anchor the embolisation device 600 within the bodily lumen L. The anchoring force provided by the plurality of flexible bristles and the flow restricting membrane may be sufficient to resist migration of the embolisation device 600 in the bodily lumen L.

In the expanded deployed configuration shown in Figure 6B, the embolisation device 600 may occlude the bodily lumen L and promote clot formation therein.

An embolisation device comprising one or more twisted portions to secure the bristles, such as that illustrated in Figures 3 and 4, may be manufactured by:
- providing a plurality of flexible bristles; and
- twisting at least one bristle of the plurality of bristles to form at least one twisted portion, the at least one twisted portion securing the plurality of bristles to one another such that the bristles extend at least radially outwardly from the at least one twisted portion.

The method may comprise forming a plurality of twisted portion. For example, the method may comprise plaiting or braiding the bristles together at a central portion.

The bristles may additionally be shape-set once the twisted portions have been formed in order to securely fasten the bristles in place.

It will be appreciated by the skilled person that there are a multitude of possible manners in which to twist the at least one bristle. The number of twisted portions and the manner in which the bristle is twisted to secure the bristles will depend on various factors such as the number of bristles to be secured, the mechanical properties of the bristles and the diameter and length of the bristles.

A core body is additionally provided after the twisted portions are formed. For example, the one or more twisted portions may be inserted into a mould and a material for the core body may be added to the mould and set. Alternatively, the core body material could be added directly to the twisted portions, for example in liquid phase, and set to create the core body.

The embolisation devices disclosed herein may further comprise a radiopaque marker attached to the embolisation device. The radiopaque marker may be the interconnecting module (for example the interconnecting module may be made of or comprise a radiopaque material) or a separate element attached to the embolisation device. The marker may be connected to the embolisation device, for example, by adhesive or welding.

## Claims

1. An embolisation device (300, 600) for promoting clot formation in a bodily lumen (L), the embolisation device (300, 600) having a collapsed delivery configuration and an expanded deployed configuration, the embolisation device (300, 600) comprising a plurality of flexible bristles (220), wherein at least one of the plurality of bristles (220) comprises a twisted portion (350), the at least one twisted portion (350) securing the plurality of bristles (220) to one another, and wherein the plurality of bristles (220) extend at least radially outwardly from the at least one twisted portion (350);
**characterised in that**
the embolisation device (300, 600) further comprises a core body (210), wherein the at least one twisted portion (350) of the bristles (220) are located within the core body (210) such that the core body (210) surrounds and secures the at least one twisted portion (350).

2. The embolisation device (300, 600) of claim 1, wherein the core body (210) mechanically anchors the plurality of flexible bristles (220).

3. The embolisation device (300, 600) of claim 1 or 2, wherein the core body (210) adheres or bonds to the plurality of flexible bristles (220).

4. The embolisation device (300, 600) of any one of claims 1 to 3, wherein the core body (210) is a curable material or settable material, and, optionally, wherein the core body (210) is curable or settable upon heating, solvent flashing and/or irradiating.

5. The embolisation device (300, 600) of any one of claims 1 to 4, wherein the core body (210) is cured or set such that the core body (210) surrounds and secures the portions of the flexible bristles (220) extending through the core body (210).

6. The embolisation device (300, 600) of any one of claims 1 to 5, wherein the core body (210) is a polymer, and, optionally, a nylon.

7. The embolisation device (300, 600) of any one of claims 1 to 6, wherein the core body (210) is a resin.

8. The embolisation device (300, 600) of any one of claims 1 to 5, wherein the core body (210) is a metal and/or an alloy.

9. An embolisation system comprising the embolisation device (300, 600) of any preceding claim, further comprising an interconnecting module (360, 550, 610) attached to the embolisation device (300, 600).

10. The embolisation system of claim 9, wherein the interconnecting module (360, 610) is configured to connect to a delivery element (620).

11. The embolisation system of claim 9, wherein the interconnecting module (550) is configured to connect to a bristle segment comprising a second plurality of flexible bristles (220).

12. The embolisation system according to any of claims 9 to 11, further comprising a radiopaque marker attached to the embolisation device (300, 600).

13. The embolisation device (300, 600) according to any of claims 1 to 8, the embolisation device (300, 600) comprising a plurality of bristle segments, each segment comprising a plurality of flexible bristles (220), wherein the twisted portion (350) secures the plurality of bristles (220) in a bristle segment to one another.

14. A method of manufacturing an embolisation device (300, 600) for promoting clot formation in a lumen (L) and having a collapsed delivery configuration and an expanded deployed configuration, the method comprising:
providing a plurality of flexible bristles (220);
twisting at least one bristle of the plurality of bristles (220) to form at least one twisted portion (350), the at least one twisted portion (350) securing the plurality of bristles (220) to one another such that the bristles (220) extend at least radially outwardly from the at least one twisted portion (350);
**characterised in that** the method comprises
providing a core body (210), wherein the at least one twisted portion (350) of the bristles (220) are located within the core body (210) such that the core body (210) surrounds and secures the at least one twisted portion (350).

## Patentansprüche

1. Embolisationsvorrichtung (300, 600) zur Förderung einer Verschlussbildung in einem Körperlumen (L), wobei die Embolisationsvorrichtung (300, 600) eine reduzierte Abgabekonfiguration und eine erweiterte eingeführte Konfiguration aufweist, wobei die Embolisationsvorrichtung (300, 600) eine Vielzahl von flexiblen Borsten (220) umfasst, wobei mindestens eine der Vielzahl von Borsten (220) einen verdrehten Abschnitt (350) umfasst, wobei der mindestens eine verdrehte Abschnitt (350) die Vielzahl von Borsten (220) aneinander befestigt, und wobei sich die Vielzahl von Borsten (220) von dem mindestens einen verdrehten Abschnitt (350) zumindest radial nach außen erstreckt;
**dadurch gekennzeichnet, dass** die Embolisationsvorrichtung (300, 600) weiter einen Kernkörper (210) umfasst, wobei sich der mindestens eine verdrehte Abschnitt (350) der Borsten (220) in dem Kernkörper (210) befindet, sodass der Kernkörper (210) den mindestens einen verdrehten Abschnitt (350) umgibt und befestigt.

2. Embolisationsvorrichtung (300, 600) nach Anspruch 1, wobei der Kernkörper (210) die Vielzahl von flexiblen Borsten (220) mechanisch verankert.

3. Embolisationsvorrichtung (300, 600) nach Anspruch 1 oder 2, wobei der Kernkörper (210) an der Vielzahl von flexiblen Borsten (220) anhaftet oder daran gebunden ist.

4. Embolisationsvorrichtung (300, 600) nach einem der Ansprüche 1 bis 3, wobei der Kernkörper (210) ein aushärtbares Material oder verfestigbares Material ist, und wobei optional der Kernkörper (210) durch Erwärmen, Lösungsmittelverdampfung und/oder Bestrahlen aushärtbar oder verfestigbar ist.

5. Embolisationsvorrichtung (300, 600) nach einem der Ansprüche 1 bis 4, wobei der Kernkörper (210) so ausgehärtet oder verfestigt wird, dass der Kernkörper (210) die Abschnitte der flexiblen Borsten (220), die sich durch den Kernkörper (210) erstrecken, umgibt und befestigt.

6. Embolisationsvorrichtung (300, 600) nach einem der Ansprüche 1 bis 5, wobei der Kernkörper (210) ein Polymer und optional ein Nylon ist.

7. Embolisationsvorrichtung (300, 600) nach einem der Ansprüche 1 bis 6, wobei der Kernkörper (210) ein Harz ist.

8. Embolisationsvorrichtung (300, 600) nach einem der Ansprüche 1 bis 5, wobei der Kernkörper (210) ein Metall und/oder eine Legierung ist.

9. Embolisationssystem, umfassend die Embolisationsvorrichtung (300, 600) nach einem der vorstehenden Ansprüche, weiter umfassend ein Verbindungsmodul (360, 550, 610), das an der Embolisationsvorrichtung (300, 600) angebracht ist.

10. Embolisationssystem nach Anspruch 9, wobei das Verbindungsmodul (360, 610) zum Verbinden mit einem Abgabeelement (620) konfiguriert ist.

11. Embolisationssystem nach Anspruch 9, wobei das Verbindungsmodul (550) zum Verbinden mit einem Borstensegment konfiguriert ist, das eine zweite Vielzahl von flexiblen Borsten (220) umfasst.

12. Embolisationssystem nach einem der Ansprüche 9 bis 11, weiter umfassend einen röntgendichten Marker, der an der Embolisationsvorrichtung (300, 600) angebracht ist.

13. Embolisationsvorrichtung (300, 600) nach einem der Ansprüche 1 bis 8, wobei die Embolisationsvorrichtung (300, 600) eine Vielzahl von Borstensegmenten umfasst, wobei jedes Segment eine Vielzahl von flexiblen Borsten (220) umfasst, wobei der verdrehte Abschnitt (350) die Vielzahl von Borsten (220) in einem Borstensegment aneinander befestigt.

14. Verfahren zur Herstellung einer Embolisationsvorrichtung (300, 600) zur Förderung einer Verschlussbildung in einem Lumen (L) und mit einer reduzierten Abgabekonfiguration und einer erweiterten eingeführten Konfiguration, wobei das Verfahren umfasst:
Bereitstellen einer Vielzahl von flexiblen Borsten (220);
Verdrehen von mindestens einer Borste der Vielzahl von Borsten (220), um mindestens einen verdrehten Abschnitt (350) zu bilden, wobei der mindestens eine verdrehte Abschnitt (350) die Vielzahl von Borsten (220) aneinander befestigt, sodass sich die Borsten (220) von dem mindestens einen verdrehten Abschnitt (350) zumindest radial nach außen erstrecken;
**dadurch gekennzeichnet, dass** das Verfahren umfasst:
Bereitstellen eines Kernkörpers (210), wobei sich der mindestens eine verdrehte Abschnitt (350) der Borsten (220) in dem Kernkörper (210) befindet, sodass der Kernkörper (210) den mindestens einen verdrehten Abschnitt (350) umgibt und befestigt.

## Revendications

1. Dispositif d'embolisation (300, 600) pour favoriser la formation de caillot dans une lumière corporelle (L), le dispositif d'embolisation (300, 600) présentant une configuration d'administration repliée et une configuration déployée étendue, le dispositif d'embolisation (300, 600) comprenant une pluralité de poils souples (220), dans lequel au moins un poil de la pluralité de poils (220) comprend une partie torsadée (350), la au moins une partie torsadée (350) fixant la pluralité de poils (220) entre eux, et dans lequel la pluralité de poils (220) s'étendent au moins radialement vers l'extérieur à partir de la au moins une partie torsadée (350) ;
**caractérisé en ce que**
le dispositif d'embolisation (300, 600) comprend en outre un corps principal (210), dans lequel la au moins une partie torsadée (350) des poils (220) sont situées à l'intérieur du corps principal (210) de sorte que le corps principal (210) entoure et fixe la au moins une partie torsadée (350).

2. Dispositif d'embolisation (300, 600) selon la revendication 1, dans lequel le corps principal (210) ancre mécaniquement la pluralité de poils souples (220).

3. Dispositif d'embolisation (300, 600) selon la revendication 1 ou 2, dans lequel le corps principal (210) adhère ou colle à la pluralité de poils souples (220).

4. Dispositif d'embolisation (300, 600) selon l'une quelconque des revendications 1 à 3, dans lequel le corps principal (210) est un matériau durcissable ou un matériau solidifiable, et, facultativement, dans lequel le corps principal (210) est durcissable ou solidifiable par chauffage, évaporation de solvant et/ou irradiation.

5. Dispositif d'embolisation (300, 600) selon l'une quelconque des revendications 1 à 4, dans lequel le corps principal (210) est durci ou solidifié de sorte que le corps principal (210) entoure et fixe les parties des poils souples (220) s'étendant à travers le corps principal (210).

6. Dispositif d'embolisation (300, 600) selon l'une quelconque des revendications 1 à 5, dans lequel le corps principal (210) est un polymère, et, facultativement, un nylon.

7. Dispositif d'embolisation (300, 600) selon l'une quelconque des revendications 1 à 6, dans lequel le corps principal (210) est une résine.

8. Dispositif d'embolisation (300, 600) selon l'une quelconque des revendications 1 à 5, dans lequel le corps principal (210) est un métal et/ou un alliage.

9. Système d'embolisation comprenant le dispositif d'embolisation (300, 600) selon une quelconque revendication précédente, comprenant en outre un module d'interconnexion (360, 550, 610) rattaché au dispositif d'embolisation (300, 600).

10. Système d'embolisation selon la revendication 9, dans lequel le module d'interconnexion (360, 610) est configuré pour se connecter à un élément d'administration (620).

11. Système d'embolisation selon la revendication 9, dans lequel le module d'interconnexion (550) est configuré pour se connecter à un segment de poils comprenant une seconde pluralité de poils flexibles (220).

12. Système d'embolisation selon l'une quelconque des revendications 9 à 11, comprenant en outre un marqueur radio-opaque rattaché au dispositif d'embolisation (300, 600).

13. Dispositif d'embolisation (300, 600) selon l'une quelconque des revendications 1 à 8, le dispositif d'embolisation (300, 600) comprenant une pluralité de segments de poils, chaque segment comprenant une pluralité de poils souples (220), dans lequel la partie torsadée (350) fixe la pluralité de poils (220) dans un segment de poils entre eux.

14. Procédé de fabrication d'un dispositif d'embolisation (300, 600) pour favoriser la formation de caillot dans une lumière (L) et présentant une configuration d'administration repliée et une configuration déployée étendue, le procédé comprenant :
le fait de prévoir une pluralité de poils souples (220) ;
le fait de torsader au moins un poil de la pluralité de poils (220) pour former au moins une partie torsadée (350), la au moins une partie torsadée (350) fixant la pluralité de poils (220) entre eux de sorte que les poils (220) s'étendent au moins radialement vers l'extérieur à partir de la au moins une partie torsadée (350) ;
**caractérisé en ce que** le procédé comprend
le fait de prévoir un corps principal (210), dans lequel la au moins une partie torsadée (350) des poils (220) sont situées à l'intérieur du corps principal (210) de sorte que le corps principal (210) entoure et fixe la au moins une partie torsadée (350).
